# EUROPÄISCHE PATENTSCHRIFT

(11) **EP 4 186 532 B1**
(45) Veröffentlichungstag und Bekanntmachung des Hinweises auf die Patenterteilung: **10.04.2024**
(21) Anmeldenummer: 21211066.2
(22) Anmeldetag: 29.11.2021
(51) Int. Cl.: A61L 15/18, A61L 15/26, A61L 15/42, A61N 1/04, B32B 33/00

(54) **ELEKTRET-BASIERTE WUNDAUFLAGE**
ELECTRET-BASED WOUND DRESSING
COMPRESSE POUR PLAIES À BASE D'ÉLECTRET

(43) Veröffentlichungstag der Anmeldung: 31.05.2023
(73) Patentinhaber: Asetreat GmbH & Co.KG, 70192 Stuttgart (DE)
(72) Erfinder: Bolinger, Walter, 07100 Sóller (ES)
(74) Vertreter: Müller-Boré & Partner Patentanwälte PartG mbB

(56) Entgegenhaltungen:
- CA-A- 864 495
- DE-A1- 2 757 103
- DE-A1-102017 101 950
- KONIKOFF J J ET AL: "Wound repair using foil electrets", CONFERENCE ON ELECTRICAL INSULATION & DIELECTRIC PHENOMENA - ANNUAL REPORT 1978, IEEE, 30. Oktober 1978 (1978-10-30), Seiten 304-310, XP032998705, DOI: 10.1109/CEIDP.1978.7728230 ISBN: 978-0-309-02861-5 [gefunden am 2016-11-01]

## Beschreibung

Die vorliegende Erfindung betrifft eine Elektret-basierte Wundauflage, insbesondere zur Verwendung bei chronischen, nicht-heilenden Wunden, umfassend ein Laminat, aufgebaut aus zwei Folien aus expandiertem gesinterten PTFE mit einer Dicke im Bereich von 0,2 bis 1 mm, wobei zwischen den beiden PTFE Folien eine Aluminiumfolie mit einer Dicke im Bereich von 4 bis 100 µm gelegt ist, wobei die elektrische Feldstärke auf mindestens einer Oberflächenseite der Wundauflage im Bereich von 100 bis 300 mV/mm eingestellt ist.

Eine effiziente Wundbehandlung ist auch heute noch eine große Herausforderung für die Medizin mit großem sozioökonomischem Effekt. Nicht nur Patienten leiden bei verzögerter Wundheilung unter Langzeitfolgen mit einer verringerten Lebensqualität, ebenso ist die finanzielle, volkswirtschaftliche Auswirkung enorm. In Europa betragen die Kosten für Wundbehandlungen ca. 4% des gesamten Gesundheitsbudgets. In den USA entfallen ca. 33% der medizinischen Kosten für die Behandlung von Diabetes auf die Therapie chronischer Wunden. Die wirtschaftliche Dimension wird deutlich, wenn man den Zuwachs an Diabetikern in den nächsten 20 Jahren berücksichtigt, und zwar mit einer weltweiten Anzahl von erwarteten Diabetespatienten von weit über einer halben Milliarde. Davon werden mindestens 15% unter chronischen Wunden leiden und eine entsprechende Therapie benötigen. Bedenkt man, dass dies nur Angaben zu einer bestimmten Wundentität sind, es aber weitaus mehr Anforderungen in der modernen Wundbehandlung gibt, so wird das wirtschaftliche Potential mehr als deutlich.

Der Prozess der Wundheilung basiert auf einer Serie von biologischen Mechanismen mit einer Vielzahl an beteiligten Zellen und biochemischen Kaskaden. Dieser Vorgang beinhaltet sowohl lokale als auch systemische Faktoren mit komplexen biomolekularen Reaktionswegen. Vereinfacht dargestellt lässt sich die Wundheilung in vier spezifische Phasen unterteilen:
1. Hämostase;
2. Inflammation;
3. Proliferation; und
4. Remodelling.

Jede dieser Phasen ist geprägt von dezidierten Zellinteraktionen und insbesondere von der sogenannten "Hautbatterie". Diese "Hautbatterie" ist ein natürlich vorkommendes elektrisches Potential mit einer Spannungsdifferenz zwischen der Epidermis und Dermis von 30 bis 100 mV. Es ist schon seit langem bekannt, dass dieses elektrische Feld eine Rolle im Rahmen von Wunden bzw. Wundheilungen spielt. So hat Du Bois-Reymond schon im Jahre 1843 einen elektrischen Strom von 1 µA an einer Schnittwunde seines Fingers gemessen. Heute weiß man, dass die mittlere elektrische Spannung im Bereich einer frischen Wunde bei ca. 100 bis 200 mV/mm liegt und mit zunehmender Heilung langsam zurückgeht. Dieses transepitheliale Potential ist das Resultat einer Akkumulation von negativen und positiven Ladungen, hervorgerufen durch eine ungleiche Verteilung von Natrium- und Kalium-Kanalfunktionen. Sobald die Hautbarriere durchbrochen wird, wie bei einer Wunde, entsteht ein elektrisches "Leck" aufgrund eines veränderten Widerstandes zwischen der durchbrochenen Barriere (Wunde) und der normalen Haut. Das daraus entstandene elektrische Potential spielt eine entscheidende Rolle im Verlauf der Wundheilung, insbesondere durch den Effekt auf die Protein-Biosynthese sowie durch eine Aktivierung der Zellmigration, die wesentlich zur Heilung einer Wunde beitragen. Wenn diese endogene Batterie Fehlfunktionen aufweist oder eine zu geringe Aktivität bzw. Ladung hat, dann kommt es zu verzögerten oder nicht heilenden und damit chronischen Wunden. Genau an diesem Punkt setzt die Entwicklung einer elektrischen Wundtherapie an. Durch eine Nachahmung der natürlichen elektrischen Funktion bzw. einer Verstärkung des elektrischen Umfeldes kann nachweislich ein positiver Effekt auf die Heilung von sowohl einfachen, als auch komplizierten Wunden erzielt werden.

Es existiert eine Vielzahl verschiedener Therapieansätze zur Wundbehandlung, manche mit invasivem Ansatz durch Debridment und Hautersatzverfahren, andere nichtinvasive Verfahren wie komprimierende Verbände, Wundauflagen mit verschiedenen Substanzen wie Alginaten etc. oder hyperbare Sauerstoffbehandlung und Unterdrucktherapie, hydrokolloide Verbände, Ultraschalltherapie sowie noch in geringerem Ausmaß auch elektrische Stimulationstherapien.

Der Effekt der elektrischen Stimulation auf verschiedene, in die Wundheilung involvierte Zellpopulationen, konnte *in vitro* gut demonstriert werden. Dabei zeigten sich Veränderungen an Makrophagen, Fibroblasten, epidermalen Zellen, Bakterien und endothelialen Zellen in Bezug auf die Migration, Proliferation, Orientierung, ein Anstieg der Proteinbiosynthese und DNA-Synthese sowie antibakterielle Effekte. Dies konnte auch in zahlreichen *in vivo* Tierexperimenten gezeigt werden, wo es zu deutlichen positiven Effekten in den unterschiedlichen Phasen der Wundheilung kam.

Elektrotherapie wird in einer Vielzahl von Erkrankungen angewendet, so zum Beispiel zur Behandlung chronischer Schmerzen, als Herzschrittmacher und zur Chochlea-Stimulation bei Hörschwierigkeiten.

Im Bereich der Wundbehandlung können derzeit vier verschiedene Therapieansätze unterschieden werden:
1. Gleichstromapplikation;
2. Wechselstromapplikation;
3. gepulste Stromapplikation; und
4. TENS (transkutane elektrische Nervenstimulation).

Dabei variieren innerhalb der Gruppen die Stromstärke und Dauer der Applikation stark, so dass vergleichende Analysen aufgrund der Heterogenität der Applikation sowie der Natur gegebenen Heterogenität von Wunden schwierig sind. Daher existieren hierzu nur wenige randomisierte, kontrollierte Studien. Fasst man die Ergebnisse dieser Studien kurz zusammen, so kann festgehalten werden, dass kurzeitig angewendete Stromapplikation die Wundheilungsraten deutlich verbessern kann und somit das "proof of principle" als positiv zu bewerten ist, so dass hier ein großes Marktpotenzial vorhanden ist.

Die exogene elektrische Stimulation wird seit einigen Jahren als Behandlungsmethode für chronische, nicht-heilende Wunden in klinischen Studien untersucht. Dabei konnte eine Aktivierung der Wundheilung, also eine Verringerung der Wundgröße, in einer Vielzahl von Studien nachgewiesen werden. Aufgrund dieser positiven Studienergebnisse wurde bereits eine Reihe von Geräten zur exogenen elektrischen Stimulation für eine beschleunigte Wundheilung entwickelt. Keines dieser Geräte wurde aber bislang von der FDA für die Anwendung zur Verbesserung der Wundheilung zugelassen. Eine beschleunigte Wundheilung wird laut klinischer Studien mit Hilfe einer Vielzahl verschiedener elektrischer Ströme wie Gleichstrom, Wechselstrom oder Impulsstrom (bzw. Kombinationen davon) und diverser Behandlungsprotokolle (Stromstärken und Behandlungszeiten) erzielt. Abgesehen von der positiven Wirkung auf die Wundheilung durch die gerichtete Zellmigration etc. hin zur Zielelektrode gibt es auch Hinweise auf eine positive Wirkung der elektrischen Stimulation auf die Wundheilung unabhängig von ihrer Polarität. Da die meisten Geräte zur exogenen elektrischen Stimulation elektrodenbasiert arbeiten, ist bei jeder Anwendung eine direkte Kontaktierung der Haut notwendig, um den Stromkreislauf herzustellen. Die Anwendung auf der Wunde birgt aber das Risiko einer Infektion und Hautreizung und stellt insofern eine technische Herausforderung dar. Die derzeit angewendeten Elektrotherapien sind daher in ihrer Anwendung mit externen, batteriegetriebenen Stromquellen oder kompliziert unflexiblen Wundauflagen noch zu umständlich und aufwändig. Somit kommen diese Therapieprinzipien nur vereinzelt zur Anwendung.

Aus dem Stand der Technik sind elektrisch aufgeladene blutstillende Wundauflagen bekannt, welche als Elektret ausgebildet sind und welche direkt auf eine Wunde gelegt werden. Durch das elektrische Feld des Elektreten, in den meisten Fällen ein polarisiertes Dielektrikum, wird die Wundheilung beschleunigt.

Ein Elektret ist ein elektrisch isolierendes Material, welches gespeicherte elektrische Ladungen oder ausgerichtete elektrische Dipole enthält und somit ein Feld in seiner Umgebung erzeugt. Elektrete, welche in industriellen Anwendungen eingesetzt werden, sind meistens aus einem Polymer gebildet. Für die aus dem Stand der Technik bekannten Elektret-Wundauflagen wird vorzugsweise Polytetrafluorethylen (PTFE) als Material verwendet, wobei dieses Material während des Herstellungsverfahrens unter hoher Temperatur und in einem starken elektrischen Feld polarisiert oder aufgeladen und abgekühlt wird. Das Feld positioniert dabei die Ladungsträger neu oder richtet die Dipole im Material aus. Wenn das Material abkühlt, behalten die Ladungsträger ihre neuen Positionen oder Orientierungen. Elektrete können jedoch auch hergestellt werden, indem Ladungen auf oder in der Nähe der Oberfläche mit Hilfe von Hochspannungs-Korona-Aufladung aufgebaut werden.

Die elektrische Feldstärke eines Elektrets zerfällt mit der Zeit exponentiell, wobei die Zerfallskonstante eine Funktion der elektrischen Materialkonstanten und der Umgebungsbedingungen ist. Materialien mit extrem hohem spezifischen Widerstand, wie z.B. PTFE, können über viele Jahre geladen bleiben. PTFE verfügt nebst hervorragenden elektrischen Eigenschaften auch über die für die Anwendung als Wundauflage nötige Biokompatibilität. Andere bekannte, für die Verwendung als Elektret-Wundauflagen geeignete Polymere sind Polyethylen und Polystyrol. Eine sanfte Wundauflage kann jedoch mit einer herkömmlichen geschälten PTFE-Folie leider nicht hergestellt werden.

US 6,087,549 B1 beschreibt einen mehrschichtigen laminierten Wundverband, umfassend eine Vielzahl von Schichten aus vorzugsweise Silber oder silberbeschichteten Fasern in einem gewebten Stoff, die sich mit Schichten aus nichtleitendem, vorzugsweise nichtmetallischem Stoff abwechseln. US 2018/085486 A1 beschreibt einen multifunktionalen Haut- oder Wundkompositverband als regenerativer Hautersatz. WO 2020/072655 A1 beschreibt einen Wundverband, umfassend ein mehrlagiges Strickgewebe, enthaltend eine erste Stricklage, eine zweite Stricklage und mindestens eine Silberionen enthaltende Verbindung enthält, wobei die erste Stricklage eine Vielzahl von ersten Garnen enthält und die Oberseite des Gewebes bildet und die zweite Stricklage mehrere Polytetrafluorethylen (PTFE)-Garne enthält und die untere Oberfläche des Gewebes bildet. CN 102335450 A, CN 111513925 A, CN 110090316 A, CN 102379776 A, CN 107714299 A und CN 102641518 A beschreiben Wundauflagen, die zum Teil mehrschichtig aufgebaut sind und zum Teil Elektret-basiert sind.

DE 27571031 A offenbart eine lektrostatische Wundauflage umfassend ein Elektret, z.B. eine Polytetrafluoroethylen-Folie mit einer Dicke von 25 µm, auf die einseitig eine 18 µm dicke Aluminiumschicht aufgedampft ist.

Ausgehend vom Stand der Technik liegt der vorliegenden Erfindung daher die Aufgabe zugrunde, vorbesagte Nachteile zu überwinden und ein elektrisch aufgeladenes wundheilendes Material bereitzustellen, dass sich anwenderfreundlich insbesondere zur Behandlung chronischer, nicht-heilender Wunden eignen soll, wobei das Material in der Anwendung keine externe, z.B. batteriebetriebene, Energiequelle einbeziehen soll. Die vorstehend beschriebene Aufgabe wird durch die in den Ansprüchen gekennzeichneten Ausführungsformen der vorliegenden Erfindung gelöst.

Gemäß der vorliegenden Erfindung wird eine Elektret-basierte Wundauflage, insbesondere zur Verwendung bei chronischen, nicht-heilenden Wunden, bereitgestellt, umfassend ein Laminat, aufgebaut aus zwei Folien aus expandiertem gesinterten PTFE mit einer Dicke im Bereich von 0,2 bis 1 mm, wobei zwischen den beiden PTFE Folien eine Aluminiumfolie mit einer Dicke im Bereich von 4 bis 100 µm gelegt ist, wobei die elektrische Feldstärke auf mindestens einer Oberflächenseite der Wundauflage im Bereich von 100 bis 300 mV/mm eingestellt ist.

Die Elektret-basierte Wundauflage gemäß der vorliegenden Erfindung braucht in der Anwendung keine externe, z.B. batteriebetriebene, Energiequelle. Erfindungsgemäß wird eine stabile Ladung auf mindestens einer Oberflächenseite der Wundauflage im Bereich von 100 bis 300 mV/mm bereitgestellt bzw. erzeugt. Es wird also erfindungsgemäß eine spezifizierte, quantitativ eingestellte, stabile elektrische Feldstärke vorgesehen. Mit einer stabilen Ladung auf mindestens einer Oberflächenseite der Wundauflage im Bereich von 100 bis 300 mV/mm ist unter anderem auch gemeint, dass bei entsprechender Sterilisation der Wundauflage und gegebenenfalls handelsüblicher Verpackung auch nach Lagerung die auf der Wundauflage spezifisch bereitgestellte Feldstärke beibehalten wird.

Polytetrafluorethylen (PTFE) hat den Vorteil, dass es hypoallergen ist, was das direkte Anlegen einer erfindungsgemäßen Wundauflage bzw. des wundheilenden Materials auf die Haut unproblematisch macht. Durch den hohen spezifischen Widerstand von PTFE können erfindungsgemäße wundheilende Materialien zudem über sehr lange Zeit geladen bleiben. Wie bereits vorstehend erläutert, werden für die Anwendung als Wundauflagen expandierte PTFE-Folien verwendet. Solche Folien sind allerdings für eine homogene Aufladung nicht geeignet. Die über das Volumen variierende Dichte von expandierten PTFE-Folien ist dabei der Hauptgrund für die beobachteten Differenzen bei der Aufladung. Jedoch hat sich überraschenderweise expandiertes gesintertes PTFE als ein weitaus vorteilhafteres Material erwiesen. Expandiertes gesintertes PTFE ist bekannt; vgl. beispielsweise DE 10 2005 036 122 A1. Die erzeugte Ladung auf und in expandierten, gesinterten PTFE-Elektreten ist aber leider üblicherweise sehr inhomogen verteilt. Das erzeugte elektrische Feld kann trotz einer konstanteren Foliendichte enorm variieren, und zwar variiert die Oberflächenspannung, d. h. das elektrische Potenzial, bei einer Herstellungsgröße von ca. 1 m² im Bereich von 5:1. Es ist aber bekannt, dass verschiedene Wundarten und Hauttypen für eine optimale Heilungsbeschleunigung Elektrete mit verschiedenen homogenen Aufladungen verlangen.

Erfindungsgemäß wird insofern ein Elektret in Sandwich-Ausführung vorgesehen, aufgebaut aus zwei expandierten gesinterten PTFE-Folien mit einer zwischengelegten Aluminiumfolie (Aluminium-Patch), wobei die elektrische Feldstärke auf mindestens einer Oberflächenseite der Wundauflage im Bereich von 100 bis 300 mV/mm eingestellt ist.

Die zwischen die zwei expandierten gesinterten PTFE-Folien gelegte Aluminiumfolie (Aluminium-Patch) ist gezielt spezifisch aufgeladen, so dass auf mindestens einer Oberflächenseite der Wundauflage, üblicherweise auf beiden Oberflächenseiten der Wundauflage, eine Feldstärke von 100 bis 300 mV/mm vorliegt.

In einer bevorzugten Ausführungsform der vorliegenden Erfindung sind die zwei expandierten gesinterten PTFE-Folien und die Aluminiumfolie zusammengeklebt. Dies ermöglicht, dass die als Sandwich ausgebildete Struktur zusammenhält.

In einer weiteren Ausführungsform der vorliegenden Erfindung ist die Aluminiumfolie kleiner als mindestens eine der zwei expandierten gesinterten PTFE-Folien. Auf diese Weise wird gewährleistet, dass ein erfindungsgemäßes Material auf eine Wunde gelegt werden kann, ohne dass die Aluminiumfolie in Kontakt mit der Haut kommt.

Die Herstellung einer solchen Wundauflage gemäß der vorliegenden Erfindung kann prinzipiell wie folgt erfolgen:
a. Anbringen einer Aluminiumfolie bzw. eines Aluminium-Patches auf eine erste expandierte gesinterte PTFE-Folie;
b. Aufladen dieser Struktur mittels eines Hochspannungsgenerators, und
c. Überdecken dieser Struktur mit einer zweiten expandierten gesinterten PTFE-Folie; und
d. gegebenenfalls Schneiden des derart aufgebauten Sandwichgebildes auf die richtigen Abmessungen des Wundauflagenmaterials.

Durch ein solches Verfahren kann ein erfindungsgemäßes wundheilendes Material einfach und günstig hergestellt werden.

Durch die Verwendung eines Hochspannungsgenerators mit einer variablen Spannungseinstellung können kurzfristig verschieden hohe Ladungen z.B. durch Kontakt- oder Influenz-Aufladung auf die jeweils eingesetzte Aluminiumfolie aufgebracht werden. Hochspannungsgeneratoren mit variablen Spannungen sind im Handel erhältlich, (z Bsp. von der Firma Eltex, Typ KNH 35, 3 - 30 kV). Für die Influenz-Aufladung werden zusätzlich Hochspannungs-, Hochgeschwindigkeits- bzw. Halbleiterschalter benötigt (z.B. HVS -Typenreihe der Firma Alaphalas).

In einer Ausführungsform des Herstellungsverfahrens (Verfahrensvariante 1) werden die Schritte in der Reihenfolge a, b, c und d ausgeführt. Damit kann gewährleistet werden, dass die zunächst gebildete Struktur frei zugänglich für das Aufladen durch direkte Kontaktaufladung ist, bevor sie mit einer zweiten expandierten gesinterten PTFE-Folie überdeckt wird.

In einer anderen Ausführungsform des Herstellungsverfahrens (Verfahrensvariante 2) werden die Schritte in der Reihenfolge a, c, b und d ausgeführt. Die Aluminiumfolie wird also zuerst mit einer zweiten expandierten gesinterten PTFE-Folie überdeckt, bevor mittels Influenzverfahren aufgeladen wird. Dadurch wird das Risiko einer unfallmäßigen Entladung der Aluminiumfolie minimiert.

In einer weiteren Ausführungsform des Herstellungsverfahrens werden die Schritte in der Reihenfolge a, c, d und b ausgeführt, wobei der Schritt b (Aufladung durch Influenzverfahren) unmittelbar vor dem Auflegen des wundheilenden Materials auf die zu behandelnde Wunde ausgeführt wird. Dadurch kann die Wundauflage unmittelbar vor dem Auflegen auf den für die Wunde optimalen Wert aufgeladen werden, was eine personalisierte Anwendung ermöglicht. Üblicherweise wird die Aufladungshöhe des wundheilenden Materials nämlich durch die zu behandelnde Wunde bestimmt. Dadurch kann die Aufladungshöhe an der zu behandelnden Wunde angepasst werden, was eine bessere Heilung ermöglicht.

Das vorstehende Herstellungsverfahren kann einen weiteren Schritt umfassen, bei welchem statische Aufladungen, wie zum Beispiel oberflächliche Reibungsaufladungen, von den expandierten gesinterten PTFE-Folie entfernt werden. Durch diesen Verfahrensschritt wird gewährleistet, dass die elektrische Feldstärke des wundheilenden Materials nicht durch eventuelle statische Aufladungen beeinflusst wird, was zu Fehlmessungen der Feldstärke führen würde. Üblicherweise wird die elektrische Feldstärke am erfindungsgemäßen wundheilenden Material gemessen. Dadurch kann das Herstellungsverfahren überprüft werden, so dass die fehlerhaften Materialien detektiert und aussortiert werden können.

In einer weiteren Ausführungsform wird die Messung der elektrischen Feldstärke des hergestellten Materials im Durchlaufverfahren durch einen Faradaykäfig mit Influenz-Elektrofeldmeter gemessen. Dadurch kann die elektrische Feldstärke bei einer Serienfertigung von erfindungsgemäßen wundheilenden Materialien einfach und verlässlich gemessen werden.

Anschließend wird das erfindungsgemäße wundheilende Material üblicherweise mittels einer Stanz- bzw. Schneidevorrichtung geschnitten. Dadurch können viele erfindungsgemäße Materialien aus einem großen Sandwichgebilde ausgestanzt oder ausgeschnitten werden.

Eine Herstellungsvorrichtung bzw. -anlage zur Erzeugung der erfindungsmäßen Wundauflagen umfasst Mittel für das Entrollen von expandierten gesinterten PTFE-Folien, eine Vorrichtung für das Anbringen der Aluminiumfolie auf eine der expandierten gesinterten PTFE-Folien, eine Aufladungsvorrichtung, Mittel für das Überdecken der Aluminiumfolie mit einer zweiten expandierten gesinterten PTFE Folie und üblicherweise eine Schneidevorrichtung, wodurch die erfindungsgemäßen wundheilenden Materialien in Serie hergestellt werden können. Die Anlage kann zusätzlich ein Entladesystem umfassen, wodurch - wie vorstehend bereits angemerkt - eventuelle statische Aufladungen, wie zum Beispiel oberflächliche Reibungsaufladungen, auf den PTFE-Folien entfernt werden können. Zudem kann die Herstellungsvorrichtung zusätzlich eine Messvorrichtung umfassen. Mittels der Messvorrichtung kann die elektrische Feldstärke der erfindungsgemäßen wundheilenden Materialien gemessen und so fehlerhafte Materialien detektiert werden.

Die Aufladungsvorrichtung kann eine Vorrichtung zur Influenzaufladung (Hochspannungsgenerator und HVS-Schalter) umfassen. Dadurch kann die Aufladung ohne direkte Kontaktaufladung erfolgen, selbst wenn mit einer zweiten PTFE-Folie überdeckt.

Im Rahmen der vorliegenden Erfindung wird auch eine Vorrichtung zur Messung der elektrischen Feldstärke eines erfindungsgemäßen wundheilenden Materials, die zwei gegenüberstehenden Influenz-Elektrofeldmeter und einen Faradaykäfig umfasst, wobei die Influenz-Elektrofeldmeter mindestens teilweise in dem Faradaykäfig sind und wobei der Faradaykäfig zwei Schlitze umfasst, durch welche das zu messende wundheilende Material in eine solche Vorrichtung zur Messung der elektrischen Feldstärke ein- und ausgeführt werden kann, bereitgestellt. Mittels einer solchen Vorrichtung zur Messung der elektrischen Feldstärke eines wundheilenden Materials kann die elektrische Feldstärke präzise und reproduzierbar gemessen werden. Insbesondere blockiert das Vorhandensein des Käfigs jeden äußeren Einfluss, der die Messung der elektrischen Feldstärke verfälschen könnte. Die Tatsache, dass der Faradaykäfig über zwei Schlitze verfügt, ermöglicht zudem eine einfache Integration in eine Serienfertigungslinie. In einer anderen Ausführungsform umfasst eine solche Vorrichtung zur Messung der elektrischen Feldstärke einen Verschiebungsmechanismus, mittels welchem die Influenzelektrofeldmeter gegenüber dem zu messenden wundheilenden Material verschiebbar sind. Dadurch kann der Abstand zwischen den Elektrofeldmetern und dem wundheilenden Material eingestellt, die elektrische Feldstärke bei diesem Abstand gemessen, und schließlich das Oberflächenpotenzial des Materials ermittelt werden. Die Messung der elektrischen Feldstärke eines erfindungsgemäßen wundheilenden Materials kann dann beispielsweise wie folgt durchgeführt werden:
1. Getaktetes Durchlaufen des wundheilenden Materials in der Vorrichtung zur Messung der elektrischen Feldstärke;
2. Einstellen des Abstands zwischen den Influenzelektrofeldmetern und dem zu messenden wundheilenden Material;
3. Messung der elektrischen Feldstärke mittels der Influenzelektrofeldmeter; und
4. Ermittlung des Oberflächenpotenzials des wundheilenden Materials.

### Kurzbeschreibung der Zeichnungen

Figur 1 zeigt eine seitliche schematische Ansicht einer bevorzugten Ausführungsform eines erfindungsgemäßen wundheilenden Materials.
Figur 2 zeigt eine schematische Draufsicht einer bevorzugten Ausführungsform des erfindungsgemäßen wundheilenden Materials.
Figur 3 zeigt eine schematische Darstellung einer ersten Ausführungsform einer Fertigungslinie zur Herstellung eines erfindungsgemäßen wundheilenden Materials. Diese Figur illustriert auch die Schritte eines ersten Verfahrens zur Herstellung eines erfindungsgemäßen wundheilenden Materials.
Figur 4 zeigt eine schematische Darstellung einer zweiten Ausführungsform einer Fertigungslinie zur Herstellung eines erfindungsgemäßen wundheilenden Materials. Diese Figur illustriert auch die Schritte eines zweiten Verfahrens zur Herstellung eines erfindungsgemäßen wundheilenden Materials.
Figur 5 zeigt eine schematische Darstellung einer Ausführungsform einer Vorrichtung zur Messung der elektrischen Feldstärke eines erfindungsgemäßen wundheilenden Materials.

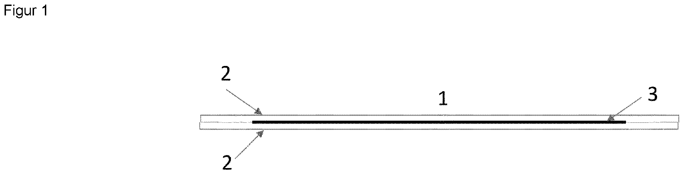

Figur 1 zeigt eine Seitenansicht einer bevorzugten Ausführungsform eines erfindungsgemäßen wundheilenden Materials (Wundauflage) 1. Das Material 1 umfasst zwei Folien 2 aus expandiertem gesinterten PTFE. Das Material 1 umfasst außerdem einen Bereich 3 aus Aluminiumfolie (Aluminium-Patch). Die Folien 2 und 3 sind in dieser bevorzugten Ausführungsform zusammengeklebt. Es wäre aber durchaus auch möglich, dass die Aluminiumfolie 3 auf eine andere Art zwischen den beiden Folien 2 gehalten wird. Das Material der Folie 3 könnte zum Beispiel aber auch direkt auf eine der Folien 3 gesputtert werden.

Erfindungsgemäß beträgt die Dicke der PTFE-Folien 0,2 bis 1 mm. Typischerweise beträgt die Dicke der Folien 2 0,4 bis 0,6 mm, vorteilhafterweise etwa 0,5 mm. Die Dicke der Aluminiumfolie 3 beträgt 4 bis 100 µm, typischerweise 5 bis 75 µm, vorzugsweise 10 bis 50 µm. Die Dicke der jeweiligen Folien 2 und 3 kann auf die spezifische Anwendung angepasst werden.

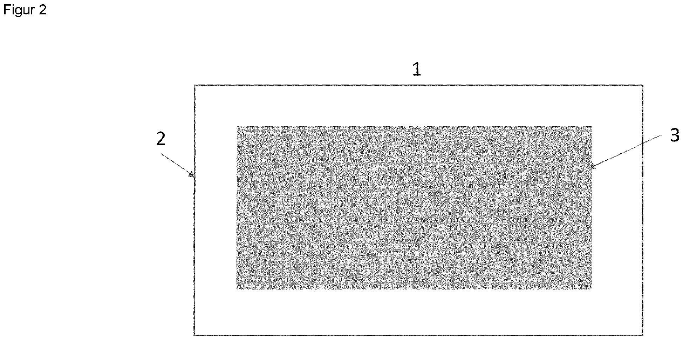

Figur 2 ist eine Draufsicht der bevorzugten Ausführungsform des erfindungsgemäßen wundheilenden Materials 1, welches in Figur 1 gezeigt ist, bei welchem die oberste Folie 2 weggelassen wurde. Wie in dieser Figur ersichtlich, ist die Aluminiumfolie 3 vorteilhafterweise von kleinerer Abmessung als die Folien 2.

Die Abmessungen des wundheilenden Materials 1 können so gewählt werden, dass dieses an die Wunde angepasst werden kann, welche es bedecken soll. Das wundheilende Material 1 ist in der Figur 2 als rechteckig dargestellt, ein Fachmann würde jedoch verstehen, dass das Material 1 irgendeine beliebige Form annehmen kann. Insbesondere kann das wundheilende Material auch die Form eines gewöhnlichen Pflasters oder einer sonstigen medizinischen Auflage aufweisen. Der Fachmann würde auch verstehen, dass die Folie 3 nicht zwingend durchgehend sein muss, und dass zwischen zwei isolierenden Folien 2 kleinere Bereiche 3 auf der Fläche der Folien 2 verteilt werden können.

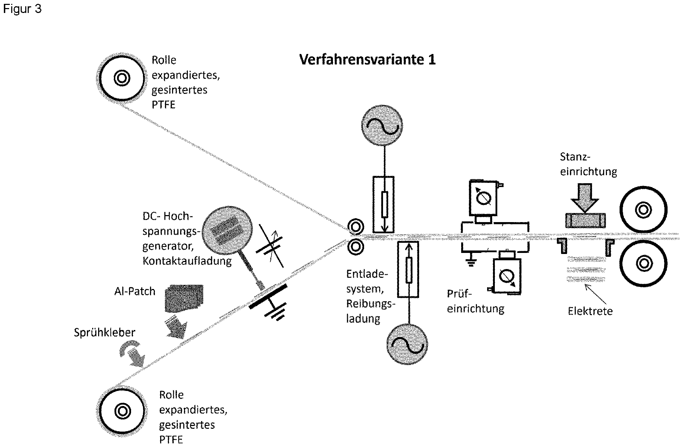

Eine erste Ausführungsform eines Verfahrens zur Herstellung eines wundheilenden Materials (Verfahrensvariante 1) ist in Figur 3 dargestellt. Die verschiedenen Schritte des Verfahrens werden mit den Buchstaben A bis G bezeichnet. Die Figur 3 dient auch als schematische Darstellung einer ersten Ausführungsform einer Vorrichtung bzw. Fertigungslinie für die Herstellung der wundheilenden Materialien 1.

In der Vorrichtung sind zwei Rollen bzw. Walzen mit aufgerolltem, expandiertem gesinterten PTFE vorgesehen. Durch die Rollen und den dazugehörigen Antriebsmitteln (hier nicht gezeigt), zum Beispiel Motoren, können aus den Rollen zwei kontinuierliche Folien gezogen werden. In einem ersten Verfahrensschritt A werden mittels einer Klebevorrichtung (Sprühkleber) beide Folien mit einem geeigneten Kleber besprüht. In einem zweiten Schritt B wird eine Aluminiumfolie, auf eine der beiden PTFE-Folien angebracht. Wie schon oben erwähnt, könnte auch eine Sputteranlage vorgesehen werden, welche ein Aluminiummetall auf eine der Folien abscheidet. Andere geeignete Methoden für das Anbringen der Aluminiumfolie sind aber auch denkbar. Ein Hochspannungsgenerator bewirkt dann in einem dritten Schritt C das elektrische Aufladen.

In der in Figur 3 illustrierten Ausführungsform erfolgt das Aufladen durch eine direkte Kontaktaufladung, bevor die zweite PTFE-Folie über die Aluminiumfolie 3 gelegt und mit der ersten PTFE-Folie verklebt wird, was dem vierten Schritt D entspricht.

In einem fünften Schritt E läuft dann das erzeugte Sandwichgebilde aus den PTFE-Folien und der dazwischen liegenden Aluminiumfolie durch ein Entladesystem durch, welches für die Entfernung von statischen Aufladungen, zum Beispiel oberflächlichen Reibungsaufladung, der PTFE-Folien vorgesehen ist. Das Entladesystem kann zum Beispiel aus einem Wechselspannungs-Entladesystem mit zwei Entladeelektroden bestehen, welche die statischen Aufladungen der Folien entlädt.

Im nächsten Schritt F kann durch eine geeignete Messvorrichtung nur diejenige elektrische Feldstärke gemessen werden, welche durch die auf dem Sandwichgebilde gespeicherten Ladungen erzeugt wird. Die Messung der elektrischen Feldstärke des Materials geschieht im Durchlaufverfahren durch einen Faradaykäfig mit zwei InfluenzElektrofeldmetern, wodurch das Oberflächenpotenzial des Materials ermittelt und somit die Qualitätskontrolle durchgeführt werden kann.

In einem siebten und letzten Schritt G wird dann das wundheilende Material 1 mittels einer Stanzvorrichtung 18 zugeschnitten, um einzelne wundheilende Produkte mit richtigen Abmessungen zu erhalten.

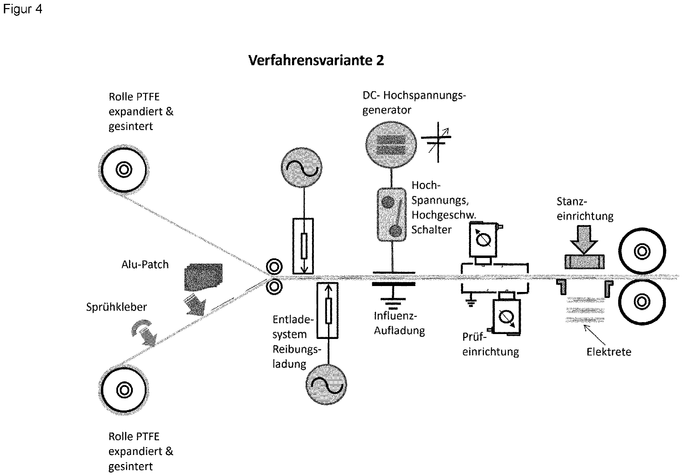

Figur 4 zeigt schematisch eine zweite Ausführungsform einer Vorrichtung 20 für die Herstellung von wundheilenden Materialien (Verfahrensvariante 2) und eines zweiten entsprechenden Herstellungsverfahrens. Die Bezeichnungen der einzelnen Verfahrensschritte wird hier beibehalten und nur deren Abfolge gemäß der zweiten Ausführungsform entsprechend angepasst.

Anders als bei der Vorrichtung und dem ersten Herstellungsverfahren (Verfahrensvariante 1) ist die Vorrichtung bzw. Fertigungsanlage dahingehend aufgebaut, dass das Sandwich-Gebilde aus den PTFE- Folien und der Aluminiumfolie schon vollständig gebildet wird, bevor im Verfahrensschritt C die Aufladung erfolgt. Nach den Schritten A, B und D, bei welchen das Sandwich-Gebilde mit den drei Schichten gebildet wird, läuft dieses durch das Entladungssystem durch, in welchem die Beseitigung von statischen Aufladungen der PTFE-Folien erfolgt. Im Schritt C wird dann die Aufladung mittels eines Hochspannungsgenerators und eines Hochgeschwindigkeitsschalters durchgeführt. Bei diesem Aufladeverfahren wird die Influenz-Aufladung angewendet. Dazu wird ein Hochspannungsstoß auf die außen angelegte Elektrode gegeben. Wie im ersten Verfahren, wird nach dem Aufladen die elektrische Feldstärke mittels einer geeigneten Messvorrichtung gemessen, und dann das wundheilende Material mittels einer Stanzvorrichtung geschnitten.

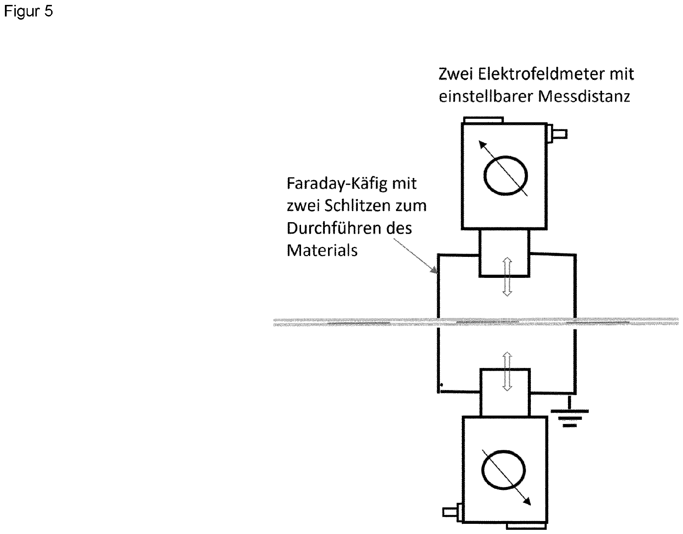

Eine bevorzugte Ausführungsform einer geeigneten Messvorrichtung für die Messung der elektrischen Feldstärke eines wundheilenden Materials ist in Figur 5 schematisch dargestellt. Die Vorrichtung umfasst zwei gegenüberstehende Influenz-Elektrofeldmeter. Die Elektrofeldmeter sind auf einem Verschiebungsmechanismus derart montiert, dass der Abstand zwischen den Messköpfen der Elektrofeldmeter und dem zu messenden wundheilenden Material präzise eingestellt und gemessen werden kann. Ferner umfasst die Vorrichtung einen Faradaykäfig, der mit der Erde verbunden ist. Der Faradaykäfig verfügt in der Regel über zwei Schlitze, durch welche das Material in die Vorrichtung ein- und ausgeführt werden kann. Durch den Faradaykäfig wird die Messung der elektrischen Feldstärke durch äußere Einflüsse nicht gestört. Die Funktionsweise eines Influenz-Elektrofeldmeters ist für einen Fachmann bekannt. Es wird deshalb darauf verzichtet, diese im Detail wiederzugeben.

## Patentansprüche

1. Elektret-basierte Wundauflage, insbesondere zur Verwendung bei chronischen, nicht-heilenden Wunden, umfassend ein Laminat, aufgebaut aus zwei Folien aus expandiertem gesinterten PTFE mit einer Dicke im Bereich von 0,2 bis 1 mm, wobei zwischen den beiden PTFE Folien eine Aluminiumfolie mit einer Dicke im Bereich von 4 bis 100 µm gelegt ist, wobei die elektrische Feldstärke auf mindestens einer Oberflächenseite der Wundauflage im Bereich von 100 bis 300 mV/mm eingestellt ist.

2. Wundauflage gemäß Anspruch 1, wobei die zwei expandierten gesinterten PTFE-Folien und die Aluminiumfolie zusammengeklebt sind.

3. Wundauflage gemäß Anspruch 1 oder 2, wobei die Aluminiumfolie kleiner als mindestens eine der zwei expandierten gesinterten PTFE-Folien ist.

4. Verfahren zur Herstellung der Wundauflage gemäß einem der Ansprüche 1 bis 3, umfassend die Schritte
a. Anbringen einer Aluminiumfolie auf eine erste expandierte gesinterte PTFE-Folie;
b. Aufladen dieser Struktur mittels eines Hochspannungsgenerators, und
c. Überdecken dieser Struktur mit einer zweiten expandierten gesinterten PTFE-Folie; und
d. gegebenenfalls Schneiden des derart aufgebauten Sandwichgebildes.

5. Verfahren gemäß Anspruch 4, wobei die Schritte in der Reihenfolge a, b, c und d ausgeführt werden.

6. Verfahren gemäß Anspruch 4, wobei die Schritte in der Reihenfolge a, c, b und d ausgeführt werden.

7. Verfahren gemäß Anspruch 4, wobei die Schritte in der Reihenfolge a, c, d und b ausgeführt werden, wobei Schritt b dann direkt vor dem Auflegen der Wundauflage durchgeführt wird, und wobei in Schritt b die Aufladung auf die für die Wunde spezifisch benötigte Feldstärke erfolgt.

## Claims

1. Electret-based wound dressing, in particular for use for chronic, non-healing wounds, comprising a laminate composed of two sheets of expanded sintered PTFE having a thickness in the range from 0.2 to 1 mm, wherein an aluminum foil having a thickness in the range from 4 to 100 µm is placed between the two PTFE sheets, wherein the electric field strength on at least one surface side of the wound dressing is set in the range from 100 to 300 mV/mm.

2. The wound dressing according to claim 1, wherein the two expanded sintered PTFE sheets and the aluminum foil are adhered together.

3. The wound dressing according to claim 1 or 2, wherein the aluminum foil is smaller than at least one of the two expanded sintered PTFE sheets.

4. A method of manufacturing the wound dressing according to any one of claims 1 to 3, comprising the steps of
a. applying an aluminum foil to a first expanded sintered PTFE sheet;
b. charging this structure by means of a high voltage generator, and
c. covering this structure with a second expanded sintered PTFE sheet; and
d. optionally cutting the sandwich structure thus formed.

5. The method according to claim 4, wherein the steps are carried out in the order a, b, c, and d.

6. The method according to claim 4, wherein the steps are carried out in the order a, c, b, and d.

7. The method according to claim 4, wherein the steps are carried out in the order a, c, d, and b, wherein step b is then carried out directly before the application of the wound dressing, and wherein in step b the charging to the field strength specifically required for the wound is carried out.

## Revendications

1. Pansement à base d'électret, notamment destiné à être utilisé pour des plaies non cicatrisantes chroniques, comprenant un stratifié composé de deux feuilles de PTFE fritté expansé ayant une épaisseur dans la plage de 0,2 à 1 mm, dans lequel une feuille d'aluminium ayant une épaisseur dans la plage de 4 à 100 µm est placée entre les deux feuilles de PTFE, dans lequel l'intensité de champ électrique sur au moins un côté de surface du pansement est réglée dans la plage de 100 à 300 mV/mm.

2. Pansement selon la revendication 1, dans lequel les deux feuilles de PTFE fritté expansé et la feuille d'aluminium sont collées ensemble.

3. Pansement selon la revendication 1 ou 2, dans lequel la feuille d'aluminium est plus petite qu'au moins une des deux feuilles de PTFE fritté expansé.

4. Procédé de fabrication du pansement selon l'une quelconque des revendications 1 à 3, comprenant les étapes consistant à
a. appliquer une feuille d'aluminium à une première feuille de PTFE fritté expansé ;
b. charger cette structure au moyen d'un générateur de haute tension, et
c. recouvrir cette structure d'une seconde feuille de PTFE fritté expansé ; et
d. découper en option la structure en sandwich ainsi formée.

5. Procédé selon la revendication 4, dans lequel les étapes sont réalisées dans l'ordre a, b, c et d.

6. Procédé selon la revendication 4, dans lequel les étapes sont réalisées dans l'ordre a, c, b et d.

7. Procédé selon la revendication 4, dans lequel les étapes sont réalisées dans l'ordre a, c, d et b, dans lequel l'étape b est ensuite réalisée directement avant l'application du pansement, et dans lequel à l'étape b, le chargement de l'intensité de champ spécifiquement requise pour le pansement est réalisé.
